# EUROPEAN PATENT SPECIFICATION

(11) **EP 3 629 772 B1**
(45) Date of publication and mention of the grant of the patent: **13.08.2025**
(21) Application number: 18810269.3
(22) Date of filing: 31.05.2018
(51) Int. Cl.: A23L 33/175, A23L 33/18, A61K 31/198, A61P 21/00, A61K 38/06

(54) **USE OF CITRULLINE AND GLUTATHIONE TO INCREASE MUSCLE MASS**
VERWENDUNG VON CITRULLIN UND GLUTATHION ZUR ERHÖHUNG DER MUSKELMASSE
UTILISATION DE LA CITRULLINE ET DU GLUTATHION POUR AUGMENTER LA MASSE MUSCULAIRE

(30) Priority: 31.05.2017 US 201762513403 P
(43) Date of publication of application: 08.04.2020
(73) Proprietor: Kyowa Hakko Bio Co., Ltd., Tokyo 100-8185 (JP)
(72) Inventor: MORITA, Masahiko, Tokyo 100-8185 (JP); KAMIMURA, Ayako, Tokyo 100-8185 (JP); WILLOUGHBY, Darryn S., Waco Texas 76798-7313 (US)
(74) Representative: Vossius & Partner Patentanwälte Rechtsanwälte mbB
(86) International application number: PCT/JP2018/020961
(87) International publication number: WO 2018/221654

(56) References cited:
- WO-A1-2017/038991
- WO-A2-01/28356
- JP-A- 2011 245 471
- US-A1- 2008 038 320
- US-A1- 2010 076 043
- US-A1- 2015 011 463
- US-A1- 2015 011 463
- US-A1- 2015 141 514
- HWANG P. ET AL.: "Eight weeks of resistance training in conjunction with glutathione and L-citrulline supplementation increases lean mass and has no adverse effects on blood clinical safety markers in resistance-trained males", J. INT. SOC. SPORTS NUTR., vol. 30, 15 January 2018 (2018-01-15), pages 1 - 10, XP055561775
- SUZUKI T. ET AL.: "The effects on plasma L-arginine levels of combined oral L-citrulline and L-arginine supplementation in healthy males", BIOSCI. BIOTECHNOL. BIOCHEM., vol. 81, no. 2, February 2017 (2017-02-01), pages 372 - 375, XP055561794

## Description

### Technical Field

The invention relates non-therapeutic method of increasing a ratio of muscle mass to total body weight in a person by administering citrulline or a salt thereof and glutathione or a salt thereof to the person in conjunction with a resistance workout program.

### Background Art

Strength training (i.e.. resistance training) is an essential component of a complete physical activity program, along with aerobic exercise. The combination of exercise helps to maintain or improve cardiorespiratory and muscular fitness. thereby contributing positively to overall health and function. Resistance training is a form of physical activity that is designed to improve muscular fitness by exercising a muscle or a muscle group against external resistance.

Increasing muscle mass, as well as maintaining muscle mass, is an important part in supporting overall physical health and wellness. For example, muscle mass plays a role in mediating metabolic health, body weight control, bone strength. and resilience to stress and disease. The benefits of maintaining or building muscle mass are well-established and include, for example, improving strength and stamina during physical activity, increasing structural stability in the body (e.g., protecting joints from injury), promoting insulin sensitivity, protecting against obesity, protecting against sarcopenia in aging, helping to preserve and maintain bone density, and improving disease recovery. In view of the many benefits of increasing muscular fitness, significant efforts have been made to better understand ways to build muscle mass whether the objective is for improving athletic performance or for improving overall health. 10004J One area of study involves the use of nutritional supplements to facilitate improved fitness. For example, understanding the role of nitric oxide (NO) in overall health is an active area of study. Although NO has a half-life of only a few seconds in the blood, NO is an important cellular signaling molecule in humans involved in many physiological and pathological processes. As such, NO and precursors of NO have long been studied in the field of medicine, particularly due to NO's action as a powerful vasodilator.

WO 01/28356 A2 relates to a food supplement comprising a substance which increases NO production in the body, and, a source of amino acids.

Vasodilation occurs during exercise as a result of various intracellular events, including the production and release of NO. NO plays an essential role in tonic and exercise-associated (e.g., exercise recovery) regulation of vasodilation and blood flow. The inventors have previously demonstrated that, in response to a single bout of resistance exercise, plasma NO. nitric oxide metabolites (NOx), and cyclic guanosine monophosphate (cGMP) are elevated 30 minutes following exercise. Without wishing to be bound to any particular theory, it is believed that there are physiological benefits of having elevated NO levels post-exercise for its impact on muscle protein metabolism and possible muscle performance in response to resistance exercise training. Similarly, NO is believed to influence skeletal muscle function through effects on excitation-contraction coupling, myofibrillar function, perfusion, and metabolism.

Citrulline is a non-proteinogenic, alpha-amino acid, which functions in vivo as an arginine precursor in arginine biosynthesis, and is a constituting factor of the NO cycle associated with NO supply. Glutathione is a tripeptide consisting of glutamic acid, cysteine, and glycine and functions to remove reactive oxygen species in vivo. Glutathione also is involved in the detoxication mechanism that removes foreign objects from the body and is thought to potentiate the effects of NO.

Despite the current understanding of nutritional supplements for improving overall fitness, there remains a continued need for methods of improving overall health and fitness.

### Summary of Invention

The invention provides a non-tnerapeutic method of increasing a ratio of muscle mass to total body weight in a person comprising administering citrulline or a salt thereof and glutathione or a salt thereof in conjunction with a resistance workout program to a person, wherein (i) the citrulline or a salt thereof and glutathione or a salt thereof are administered daily for a period greater than 7 days, (ii) the resistance workout program is administered for at least the entire period during which the citrulline or a salt thereof and glutathione or a salt thereof are administered, and (iii) the muscle mass is measured by a body composition meter.

Preferred embodiments of the present invention are also set out in the appended claims.

Moreover, it is to be noted that the below passages which refer to methods of the invention relate to non-therapeutic methods.

### Brief Description of Drawings

[fig.1A] Fig. 1A is a bar graph illustrating the change in muscle mass at 4 weeks following heavy resistance training and supplementation with a placebo (control), citrulline malate (CIT-malate) only, and glutathione (GSH) plus citrulline (CIT).
[fig.1B] Fig. 1B is a bar graph illustrating the change in muscle mass at 8 weeks following heavy resistance training and supplementation with a placebo (control), citrulline malate (CIT-malate) only, and glutathione (GSH) plus citrulline (CIT).
[fig.2]Fig. 2 is a bar graph illustrating the rate (%) of the subjects whose muscle mass consistently increased at both 4 and 8 weeks as described in the Examples following heavy resistance training and supplementation with a placebo (control), CIT-malate only, and GSH and CIT.
[fig.3A] Fig. 3A is a graph illustrating the relation of changes in muscle mass with 1-repetition maximum (1-RM) bench press after 4 weeks.
[fig.3B] Fig. 3B is a graph illustrating the relation of changes in muscle mass with 1-repetition maximum (1-RM) bench press after 8 weeks.
[fig.3C] Fig. 3C is a graph illustrating the relation of changes in muscle mass with 1-repetition maximum (1-RM) leg press after 4 weeks.
[fig.3D] Fig. 3D is a graph illustrating the relation of changes in muscle mass with 1-repetition maximum (1-RM) leg press after 8 weeks.
[fig.3E] Fig. 3E is a graph illustrating the relation of changes in muscle mass with 1-repetition maximum (1-RM) bench press in the placebo group.
[fig.3F] Fig. 3F is a graph illustrating the relation of changes in muscle mass with 1-repetition maximum (1-RM) bench press in the CIT-malate group.
[fig.3G]Fig. 3G is a graph illustrating the relation of changes in muscle mass with 1-repetition maximum (1-RM) bench press in GSH + CIT group.

### Description of Embodiments

The invention provides a non-therapeutic method of increasing a ratio of muscle mass to total body weight in a person comprising, consisting essentially of, or consisting of administering citrulline or a salt thereof and glutathione or a salt thereof, in conjunction with a resistance workout program, to a person. In the method, the citrulline or a salt thereof and glutathione or a salt thereof are administered daily for a period greater than 7 days, and the resistance workout program is administered for at least the entire period during which the citrulline or a salt thereof and glutathione or a salt thereof are administered. Muscle mass is measured by a body composition meter. Thus, the invention provides citrulline or a salt thereof and glutathione or a salt thereof for a non-therapeutic use in increasing a ratio of muscle mass to total body weight in a person in conjunction with a resistance workout program, wherein the citrulline or a salt thereof and glutathione or a salt thereof are for daily use for a period greater than 7 days, the resistance workout program lasts for at least the entire period of use of the citrulline or a salt thereof and glutathione or a salt thereof, and the muscle mass is measured by a body composition meter.

As used herein. "muscle mass" refers to the weight of the muscles in a body (e.g., in kilograms or pounds), and includes smooth muscles, skeletal muscles, as well as the water contained in the muscles. As used herein, the phrase "the two compounds" refers to citrulline or a salt thereof and glutathione or a salt thereof, unless otherwise specified. As used herein the term "about" typically refers to ± 1% of a value. ± 5% of a value, or ± 10% of a value.

The citrulline or a salt thereof can be administered in any suitable amount (e.g., dosage). If the amount of citrulline or salt thereof administered is too low, a desirable increase in the ratio of muscle mass to total body weight may not be observed. In contrast, if the amount of citrulline or salt thereof administered is too high, the method may not be cost effective or the composition comprising citrulline or salt thereof may exhibit undesirable properties (e.g.. lack of stability, poor solubility, poor taste, etc.). Accordingly, the amount of citrulline or salt thereof administered can be about 5 g/day or less, for example, about 4.5 g/day or less, about 4 g/day or less, about 3.5 g/day or less, about 3 g/day or less, or about 2.5 g/day or less. Alternatively, or in addition, the amount of citrulline or salt thereof administered can be about 50 mg/day or more, for example, about 100 mg/day or more, about 150 mg/day or more, about 200 mg/day or more, about 250 mg/day or more, about 300 mg/day or more, about 350 mg/day or more, about 400 mg/day or more, about 450 mg/day or more, about 500 mg/day or more, about 550 mg/day or more, about 600 mg/day or more, about 650 mg/day or more, about 700 mg/day or more, about 750 mg/day or more, about 800 mg/day or more, about 850 mg/day or more, about 900 mg/day or more, about 950 mg/day or more, about 1 g/day or more, about 1.1 g/day or more, about 1.2 g/day or more, about 1.3 g/day or more, about 1.4 g/day or more, about 1.5 g/day or more, about 1.6 g/day or more, about 1.7 g/day or more, about 1.8 g/day or more, about 1.9 g/day or more, or about 2 g/day or more. Thus, any two of the aforementioned endpoints can be used to define a close-ended range or can be used singly to define an open-ended range. For example, the amount of citrulline or salt thereof administered can be about 50 mg/day to about 5 g/day, about 100 mg/day to about 4.5 g/day, about 150 mg/day to about 4 g/ day, about 200 mg/day to about 3.5 g/day, about 250 mg/day to about 3 g/day, about 300 mg/day to about 2.5 g/day, about 350 mg/day to about 2 g/day, about 400 mg/day to about 1.9 g/day, about 450 mg/day to about 1.8 g/day, about 500 mg/day to about 1.7 g/day, about 550 mg/day to about 1.6 g/day, about 600 mg/day to about 1.5 g/day, about 650 mg/day to about 1.4 g/day, about 700 mg/day to about 1.3 g/day, about 750 mg/day to about 1.2 g/day, about 800 mg/day to about 1.1 g/day, about 850 mg/day to about 1g/day, or about 900 mg/day to about 950 mg/day.

In an embodiment, the amount of citrulline or salt thereof administered is about 300 mg/day or more. In another embodiment, the amount of citrulline or salt thereof administered is about 1 g/day or more. In yet another embodiment, the amount of citrulline or salt thereof administered is about 2 g/day or more.

Glutathione or its salt can be administered in any suitable amount or dosage to a person. If the amount of glutathione or salt thereof administered is too low, an increase in the ratio of muscle mass to total body weight may not be observed in the person. In contrast, if the amount of glutathione or salt thereof administered is too high, the method may not be cost effective or the composition comprising glutathione or salt thereof may exhibit undesirable properties (e.g., lack of stability, poor solubility, poor taste, etc.). Accordingly, the amount of glutathione or salt thereof administered to a person can be about 1 g/day or less, for example, about 900 mg/day or less, about 800 mg/day or less, about 700 mg/day or less, about 600 mg/day or less, or about 500 mg/ day or less. Alternatively, or in addition, the amount of glutathione or salt thereof administered can be about 25 mg/day or more, for example, about 50 mg/day or more, about 100 mg/day or more, about 150 mg/day or more, about 200 mg/day or more, about 250 mg/day or more, about 300 mg/day or more, about 350 mg/day or more, about 400 mg/day or more, or about 450 mg/day or more. Thus, any two of the aforementioned endpoints can be used to define a close-ended range or can be used singly to define an open-ended range. For example, the amount of glutathione or salt thereof administered can be about 25 mg/day to about 1 g/day, about 50 mg/day to about 900 mg/day, about 100 mg/day to about 800 mg/day, about 150 mg/day to about 700 mg/ day, about 200 mg/day to about 600 mg/day, about 250 mg/day to about 500 mg/day, about 300 mg/day to about 450 mg/day, or about 350 mg/day to about 400 mg/day.

In an embodiment, the amount of glutathione or salt thereof administered is about 50 mg/day or more. In another embodiment, the amount of glutathione or salt thereof administered is about 100 mg/day or more. In yet another embodiment, the amount of glutathione or salt thereof administered is about 200 mg/day or more.

In one embodiment of the invention, the citrulline or a salt thereof and glutathione or a salt thereof are administered to a person in any suitable ratio. Without wishing to be bound to any particular theory, it is believed that the citrulline or salt thereof increases NO concentration by acting as a precursor for the amino acid arginine. It is also believed that glutathione or its salt acts to potentiate the effects of NO by increasing its half-life. The inventors discovered that the relative amounts of each of citrulline or a salt thereof and glutathione or a salt thereof can be adjusted, as appropriate, to obtain the desired increase in ratio of muscle mass to total body weight in accordance with the inventive method. Typically, the citrulline or a salt thereof and glutathione or a salt thereof are administered each day in a molar ratio of the active agents of citrulline to glutathione of about 50:1 to about 1:1. In some embodiments, the citrulline or a salt thereof and glutathione or a salt thereof are administered each day in a molar ratio of the active agents of citrulline to glutathione of about 25:1 to about 1:1. In other embodiments, the citrulline or a salt thereof and glutathione or a salt thereof are administered each day in a molar ratio of the active agents of citrulline to glutathione of about 10:1 to about 1:1. In yet other embodiments, the citrulline or a salt thereof and glutathione or a salt thereof are administered each day in a molar ratio of the active agents of citrulline to glutathione of about 10:1.

In addition, the citrulline or a salt thereof and glutathione or a salt thereof can be administered to a person in any suitable weight ratio. Typically, the weight ratio of citrulline to glutathione is about 0.05:1 to about 200:1. for example, about 0.1:1 to about 150:1, about 0.5:1 to about 100:1, about 1:1 to about 50:1, about 2:1 to about 25:1. about 5:1 to about 15:1. or about 7:1 to about 12:1. In a preferred embodiment, the two compounds are administered in amounts such that the weight ratio of citrulline to glutathione is about 10:1.

As understood herein, referenced amounts of citrulline and/or glutathione typically refer to the amount of active species (i.e., citrulline and/or glutathione), regardless of a particular salt that may be used.

The citrulline or a salt thereof and glutathione or a salt thereof can be administered in any suitable manner to a person. For example, in some embodiments the two compounds are administered essentially at the same time (i.c.. concurrently). In addition, the two compounds can be administered with or without food, and the two compounds can be suitably administered at any time of the day or night.

As described herein, the two compounds are administered in conjunction with a resistance workout. Accordingly, the two compounds can be administered before or after a resistance workout on a particular day. Alternatively, the two compounds can be administered at different times, for example, one of the two compounds can be administered prior to a resistance workout on a particular day and the other of the two compounds can be administered after the resistance workout on the same day. In embodiments in which the two compounds are administered at different times on the same day, the order in which the compounds are administered is not particularly limiting. Further, in embodiments wherein the two compounds are administered at different times on the same day, typically the citrulline or salt thereof and glutathione and salt thereof are administered within about 3 hours or less (e.g., within about 2 hours, within about 1 hour, within about 30 minutes) of each other.

In a preferred embodiment, the two compounds are taken concurrently about one hour prior to a resistance workout on days that a resistance workout is performed as part of the resistance workout program. On days which a resistance workout is not performed, the two compounds are preferably administered in the morning (e.g., at breakfast) and/or shortly upon waking after 5 or more hours of sleep.

The method of the invention comprises administering citrulline or a salt thereof and glutathione or a salt thereof for a period of time greater than 7 days (i.c.. greater than about 168 hours), which includes about 8 days or more, about 9 days or more, about 10 days or more, about 11 days or more, about 12 days or more, and about 13 days or more. The period of administering citrulline or a salt thereof and glutathione or a salt thereof typically corresponds to a resistance workout program as described herein.

In a preferred embodiment, citrulline or a salt thereof and glutathione or a salt thereof is administered for a period of at least 14 days, i.e., at least 336 hours (e.g.. about 15 days or more, about 16 days or more, about 17 days or more, about 18 days or more, about 19 days or more, and about 20 days or more).

In another preferred embodiment, citrulline or a salt thereof and glutathione or a salt thereof is administered for a period of at least 21 days, i.e., at least 504 hours (c.g.. about 22 days or more, about 23 days or more, about 24 days or more, about 25 days or more, about 26 days or more, and about 27 days or more).

In yet another embodiment, citrulline or a salt thereof and glutathione or a salt thereof is administered for a period of at least 28 days. i.e., at least 672 hours (e.g., about 29 days or more, about 30 days or more, about 31 days or more, about 32 days or more, about 33 days or more, and about 34 days or more).

In a particularly preferred embodiment, citrulline or a salt thereof and glutathione or a salt thereof are administered for a period of 4 weeks to 8 weeks (c.g., 4 weeks. 5 weeks. 6 weeks. 7 weeks, or 8 weeks). In other embodiments, the two compounds are administered for a period of greater than 8 weeks.

The citrulline or salt thereof and glutathione or salt thereof can be administered in any suitable form, for example, any suitable stereoisomer or salt, including any suitable diastereomeric salt pair. The L-citrulline and D-citrulline stereoisomers are commercially available from several sources including, for example, Sigma-Aldrich Corporation (St. Louis. MO). Glutathione is also commercially available.

In an embodiment of the inventive method, a salt of citrulline and/or a salt of glutathione can be used, as appropriate. Suitable salts include, for example, acid addition salts, metal salts, ammonium salts, organic amine addition salts, amino acid addition salts, and the like. Suitable acid addition salts include, for example, inorganic acid salts such as hydrochlorides, sulfates, nitrates, and phosphates, and organic acid salts such as acetates, maleates, fumarates, citrates, malates, lactates, alpha-ketoglutarates, gluconates, caprylates, adipates, succinates, tartrates, ascorbates, and the like. Suitable metal salts include, for example, alkali metal salts such as sodium salts and potassium salts, alkaline earth metal salts such as magnesium salts and calcium salts, aluminum salts, zinc salts, and the like. Suitable ammonium salts include, for example, salts of ammonium, tetramethylammonium and the like. Suitable organic amine addition salts include, for example, salts of morpholine, piperidine, and the like. Suitable amino acid addition salts include, for example, salts of glycine, phenylalanine, lysine, aspartic acid, glutamic acid, and the like. In some embodiments, one or more combinations of the aforementioned salts can be administered, as appropriate.

In a particularly preferred embodiment, the citrulline salt is citrulline malate.

The methods described herein comprise administering citrulline or a salt thereof and glutathione or a salt thereof in the form of a pharmaceutical composition. In particular, a pharmaceutical composition will comprise citrulline or a salt thereof, glutathione or a salt thereof, and a pharmaceutically acceptable carrier. Suitable pharmaceutically acceptable carriers include, for example, excipients, vehicles, adjuvants, and diluents, which are well known to those who are skilled in the art and which are readily available to the public. Typically, the pharmaceutically acceptable carrier is one that is chemically inert to the active compounds and one that has no detrimental side effects or toxicity under the conditions of use.

The pharmaceutical composition can be administered using any suitable formulation, but in a preferred embodiment, the formulation is administered orally (i.e.. oral dosage form). Illustrative oral dosage forms include, for example, a capsule, pill, caplet, tablet, lozenge, troche, powder, liquid, gel, solution, suspension, quick dissolving film, and the like.

In particular, suitable oral formulations include (a) liquid solutions, such as effective amounts of citrulline or a salt thereof and glutathione or a salt thereof dissolved in a diluent, such as water, saline, or juice, and can include an additive, such as cyclodextrin (c.g.. alpha-, beta-, or gamma-cyclodextrin, hydroxypropyl cyclodextrin) or polyethylene glycol (c.g.. PEG400); (b) capsules, caplets, pills, tablets, lozenges, and troches, each containing predetermined amounts of citrulline or a salt thereof and glutathione or a salt thereof, as solids or granules; (c) powders; (d) suspensions in an appropriate liquid; and (c) suitable emulsions and gels.

Liquid formulations may include diluents, such as oil, water, alcohol (e.g., ethanol, benzyl alcohol, and the polyethylene alcohols), and other beverages, either with or without the addition of a pharmaceutically acceptable surfactant, suspending agent, or cmulsifying agent. Other beverages include fruit juice, vegetable juice, carbonated drinks (e.g., soda, club soda), coffee, tea, milk, a sport's drink, and other nutritional supplement drinks.

Capsule forms can be of the ordinary hard- or soft-shelled gelatin type containing, for example, surfactants, lubricants, and inert fillers, such as lactose, sucrose, calcium phosphate, and cornstarch.

Tablet forms can include one or more of lactose, sucrose, mannitol, corn starch, potato starch, alginic acid, microcrystalline cellulose, acacia, gelatin, guar gum, colloidal silicon dioxide, croscarmellose sodium, talc, magnesium stearate, calcium stearate, zinc stearate, stearic acid, and other excipients, colorants, diluents, buffering agents, disintegrating agents, moistening agents, preservatives, flavoring agents, and pharmacologically compatible carriers.

Lozenge forms can comprise the active ingredients in a flavor, usually sucrose and acacia or tragacanth, as well as pastilles comprising the active ingredients in an inert base, such as gelatin and glycerin, or sucrose and acacia, emulsions, gels, and the like containing, in addition to the active ingredient, such carriers as are known in the art.

In some embodiments, the method includes administering a composition comprising, consisting essentially of, or consisting of citrulline or a salt thereof, glutathione or a salt thereof, a carrier, and one or more optional additives. The additives can be selected from a solubilizer, surfactant, stabilizer, thickener, lubricant, preservative, dye (natural or synthetic), sweetener (natural or artificial), flavoring (natural or artificial), an acidulant, clectrolyte (c.g.. Na, K. and Mg compounds), and/or vitamin (e.g., vitamin A, B6. B12, C. D. E, and K, thiamine, riboflavin, niacin, folate, and biotin). Suitable solubilizers include, for example, glycerol, cyclodextrin, and polyethylene glycol. Suitable surfactants include, for example, carbomer, polysorbate 20, polysorbate 80, a polyethylene oxide/polypropylene oxide block copolymer, polyethoxylated castor oil, phospholipid, sodium lauryl sulfate, and combinations thereof, Suitable thickeners include, for example, a starch, a hydrolyzed starch, pre-gelatinized starch, a gum, or carboxymcthylcellulose. Suitable lubricants include, for example, polyethylene glycol, magnesium stearate, stearic acid, magnesium and calcium stearates, sodium stearyl fumarate, and combinations thereof, Suitable preservatives include, for example, sodium benzoate, benzyl alcohol, a paraben, potassium sorbate, chlorhexidine acetate, and combinations thereof. Suitable acidulants include, for example, citric acid, malic acid, ascorbic acid, tartaric acid, fumaric acid, and succinic acid.

In an embodiment of the inventive method, both citrulline or a salt thereof and glutathione or a salt thereof are administered in conjunction with a resistance workout program to a person to increase the ratio of muscle mass to total body weight in the person. The resistance workout program is practiced by the person for at least the entire period during which the citrulline or a salt thereof and glutathione or a salt thereof is administered.

Resistance training is commonly referred to as strength training or weight training. As used herein. "resistance workout program" refers to a system of physical conditioning introducing resistance to an exercising body. Resistance may be provided in any suitable manner including, for example, traditional free weights and dumbbells, weight machines, body weight (c.g.. yoga, pilates, plyometrics, and the like), environmental resistance (c.g.. water, air, and the like), elastic tubing, medicine balls, or even common household objects (c.g.. milk jugs filled with sand, soup cans, and the like). The resistance workout program can comprise any suitable regular resistance exercise or resistance training.

The person that is the subject of the inventive method, is typically a person who regularly trains, i.e.. does resistance training of the upper body, the lower body, or both the upper and lower body from about six months to about a year, three times a week or more. For example, in some embodiments, the person of the inventive method has participated in regular resistance training at least 3 times a week for one year prior to being subject to the inventive method.

In an embodiment of the inventive method, the subject of the inventive method is considered to be in good health by a physician and can participate in resistance training as described herein. Thus, the person of the invention typically is not substantially limited in being able to participate in a resistance training workout program. In an embodiment, the person has not been diagnosed with a disease, such as hypertension, heart failure, angina pectoris, cerebral infarction, coronary heart disease, cerebral ischemic disease induced by vascular spasm, myocardial ischemic disease, shock, renal ischemia, renal dysfunction due to renal vascular spasm, peripheral vascular spasmodic disease, cerebral hemorrhage, psoriasis, an ulcer, hepatic ischemia, intestinal ischemia, and an ischemic disease of the central nervous system. In an embodiment, the person has not been diagnosed with a metabolic disorder (c.g.. heart disease, arrhythmias, diabetes, thyroid disease, etc.), does not have a history of pulmonary disease, hypertension, autoimmune disease, cancer, peptic ulcers, or anemia, does not take any blood thinning, heart, pulmonary, thyroid, anti-hyperlipidemic, hypoglycemic, anti-hypertensive, psychotropic, neuromuscular and/or neurological, or androgenic medications, does not have a bleeding disorder, and/or does not have any chronic infections (e.g.. human immunodeficiency virus (HIV)).

The person can be male or female, but, in a preferred embodiment, the person is male. In some embodiments, the person is male and from the age of about 18 years to about 35 years.

In another preferred embodiment, the person trains continuously (e.g., 3 times a week for about 6 months or more) at the resistance strength of 70-80% of 1-repetition maximum (1-RM).

Typical resistance exercises that can be performed using free-weights, machines, or body weight include, for example, supine bench press, seated chest press, push-ups, bent-over barbell rows, lat pulldowns, pull-ups, dumbbell lateral raise, shoulder press, arm circles, barbell/dumbbell curls, cable curls, reverse grip pull-ups, dumbbell kickbacks, pressdowns, dips, weighted crunches, seated "abs" machine, crunches, prone planks, back squats, leg extension, body weight lunges, stiff-leg deadlifts, leg curls, hip-ups, and the like.

Typical upper body exercises include, for example, bench press, lat pull, shoulder press, seated row, shoulder shrugs, chest fly, biceps curl, triceps press down, and abdominal curls, and typical lower body exercises include, for example, leg press, back extension, step-ups, leg curls, leg extensions, heel raises, and abdominal crunches.

The resistance workout program of the inventive method can comprise one or more of any suitable resistance excrcise(s), as appropriate. For example, one or more resistance exercises can be performed or combined to create a resistance workout program having any suitable structure.

By way of example, according to guidelines for resistance training from the American College of Sports Medicine (ACSM) published in 2013. is recommended that a strength training program should be performed a minimum of two non-consecutive days each week, with one set of 8 to 12 repetitions for healthy adults or 10 to 15 repetitions for older or frail individuals. The ACSM recommends that 8 to 10 exercises should be performed that target the major muscle groups (e.g., muscle groups of the upper body and/or lower body).

The resistance workout program of the inventive method can be performed in conjunction with aerobic exercise. Examples of suitable aerobic exercise are known to persons of ordinary skill in the art.

In an embodiment, the resistance workout program comprises at least four weeks of twice per week training, wherein each training session includes at least one resistance exercise comprising 3 sets of 10 repetitions, with a rest period between each set, and wherein a set does not last longer than 2 minutes.

In an embodiment, the resistance workout program comprises a daily resistance workout 4 days per week, and wherein the daily resistance workout comprises at least 8 repetitions of at least one exercise that targets a muscle of interest (e.g., chest, back, shoulders, biceps, triceps, abdomen, quadriceps, hamstrings, and combinations thereof).

In other embodiments, the resistance workout program is a 4 day per week resistance training program split into two upper and two lower extremity workouts per week for a total of 8 weeks, wherein a person performs 3 sets of 10 repetitions with as much weight as he/she can lift per set (typically 70-80% of 1-RM) with rest periods between exercises and sets lasting no longer than 2 minutes.

In an embodiment of the inventive method, the resistance workout program is conducted in conjunction with or concurrently with the administration of citrulline or salt thereof and glutathione or salt thereof. Accordingly, the resistance workout program of the invention is performed for a period of greater than 7 days (e.g., at least 14 days, at least 21 days, at least 28 days, at least 5 weeks, at least 6 weeks, at least 7 weeks, or at least 8 weeks etc.).

The invention provides a method of increasing muscle mass in a person. More particularly, the invention provides a method for increasing a ratio of muscle mass to total body weight compared to the situation wherein the person is not administered citrulline or salt thereof and glutathione or salt thereof, even if the person regularly trains, as described herein. Without wishing to be bound to any particular theory, it is believed that the combination of administering citrulline or a salt thereof and glutathione or a salt thereof, in conjunction with a resistance workout, provides a physiological benefit on muscle protein metabolism and muscle performance in response to resistance training.

In an embodiment, the ratio of muscle mass to total body weight is increased by about 0.3% or more (c.g.. 0.4% or more. 0.5% or more. 0.6% or more, 0.7% or more. 0.8% or more. 0.9% or more, 1% or more, 1.1% or more. 1.2% or more. 1.3% or more. 1.4% or more. 1.5% or more. 1.6% or more. 1.7% or more, 1.8% or more. 1.9% or more, or 2% or more). In another embodiment, the ratio of muscle mass to total body weight is increased by about 0.6% or more. In yet another embodiment, the ratio of muscle mass to total body weight is increased by about 1% or more.

The muscle mass is measured using a body composition meter. Suitable body composition meters are commercially available from, for example, Hologic. Inc. (Marlborough. MA) and Tanita, Inc. (Arlington Heights. IL). Other illustrative techniques suitable for measuring muscle mass include, girth measurements, calculation methods, 24h urinary creatinine method, body scanning, and the like. In addition, other methods for measuring muscle mass include Dual-Energy X-Ray Absorptiometry (DEXA), which measures body composition including non-fat soft tissue; Total Body Potassium (TBK), which measures the body's total cell mass (that is, the active growing tissues in the body), which in turn can be used to estimate fat-free or lean body mass such that, when this measurement is combined with measurements from the Total Body Protein, total organ mass and muscle mass can be determined: Magnetic Resonance Imaging (MRI), which can be used to measure the composition of body tissue, by identifying muscle, fat and organs etc.; Total Body Electrical Conductivity (TOBEC), which can be used to estimate lean body mass; and Computed Tomography (CT), the high quality images of which can be used to differentiate and measure the amounts of fat and lean body tissue.

### Examples

This example further illustrates the invention but, of course, should not be construed as in any way limiting its scope.

The following abbreviations are used in the example: BMI refers to body mass index; ACSM refers to the American College of Sports Medicine: RM refers to repetition maximum: CIT refers to citrulline; GSH refers to glutathione; PLC refers to cellulose placebo; BUN refers to blood urea nitrogen: AST refers aspartate aminotransferase; ALT refers to alanine aminotransferase; CK refers to creatine kinase; LDH refers to lactate dehydrogenase; GGT refers to gamma-glutamyl transferase: HDL refers to high density lipoprotein; LDL refers to low density lipoprotein; MCV refers to mean corpuscular volume. MCH refers to mean corpuscular hemoglobin; MCHC refers to mean corpuscular hemoglobin per cell: RDW refers to red cell distribution width: SEM refers to standard error of the mean; and ANOVA refers to analysis of variance.

This example demonstrates the effects of an 8-week resistance training program in conjunction with daily, orally-delivered L-citrulline and glutathione, L-citrulline-malate, or placebo supplementation on body composition (c.g.. muscle mass) and whole blood and serum clinical chemistry markers in accordance with an embodiment of the invention.

Seventy-five resistance-trained (c.g.. regular, resistance training thrice weekly for at least one year prior to the study) males between the ages of 18-35 and having a BMI between 18.5 and 30 kg/m² were divided into 3 groups of 25. wherein each group received one of the following supplements: (A) 2 g/day L-citrulline (CIT) and 200 mg/ day glutathione (GSH), (B) 2 g/day L-citrulline malate only. or (C) 2.52 g/day of cellulose placebo. Participants were matched by total body mass and then randomly assigned a supplementation protocol in a double-blind fashion. Participants took their supplements one hour prior to their workout on exercise days. On non-exercise days, participants took their supplements in the morning with breakfast.

Participants met the following criteria: participants were considered low risk for cardiovascular disease with no contraindications to exercise as determined by the ACSM; participants had not consumed any nutritional supplements (other than multi-vitamins) or anabolic steroids for 3 months prior to the study; participants were free from orthopedic problems that would inhibit participant from upper- and lower-body resistance training exercises; and participants were non-smokers.

Participants also underwent a medical screening to assess their health. The medical screening was used to determine that (a) cach participant was free of metabolic disorders (c.g., heart disease, arrhythmias, diabetes, thyroid disease, etc.), (b) each participant did not have a history of pulmonary disease, hypertension, autoimmune disease, cancer, peptic ulcers, or anemia; were not taking any blood thinning, heart, pulmonary, thyroid, anti-hyperlipidemic, hypoglycemic, anti-hypertensive, psychotropic, neuromuscular and/or neurological, or androgenic medications, (c) each participant did not have any bleeding disorders, (d) each participant did not have any chronic infections (e.g., HIV), and (e) each participant did not have a known allergic reaction to topical anesthetics.

The diets of the participants were not standardized, and test subjects were asked not to change their dietary habits during the course of the study. However, the participants recorded their dietary intake for 4 consecutive days prior to the three testing sessions at days 0, 29. and 57. The 4-day dietary recalls were evaluated with the Food Processor dietary assessment software program (ESHA Research, Salem, OR) to determine the average daily macronutrient consumption of fat, carbohydrate, and protein in the diet for the duration of the study. A summary of the participants' dietary compositions is set forth in Table 1.

The experimental data were subjected to statistical analysis as described herein. Data were presented as means ± SEM. Following a comparison of the data by analysis of variance (ANOVA), either the Bonferroni correction for multiple tests or Scheffe's test for multiple comparisons was used to identify the differences among treatments. Statistical comparisons between the baseline and sequential values were analyzed using Bonferroni's test for multiple comparisons. Correlation among lean mass and muscle strength was analyzed with Peason's correlation coefficient test. A p-value of less than 0.05 was considered to indicate significance. Statistical analysis was performed with Stateel software for Windows (Version 2, OMS Publishing. Inc. Saitama, Japan) and the ystat 2000 Statistical Program File (Igaku Tosho Shuppan, Tokyo, Japan).

### [Table 1]

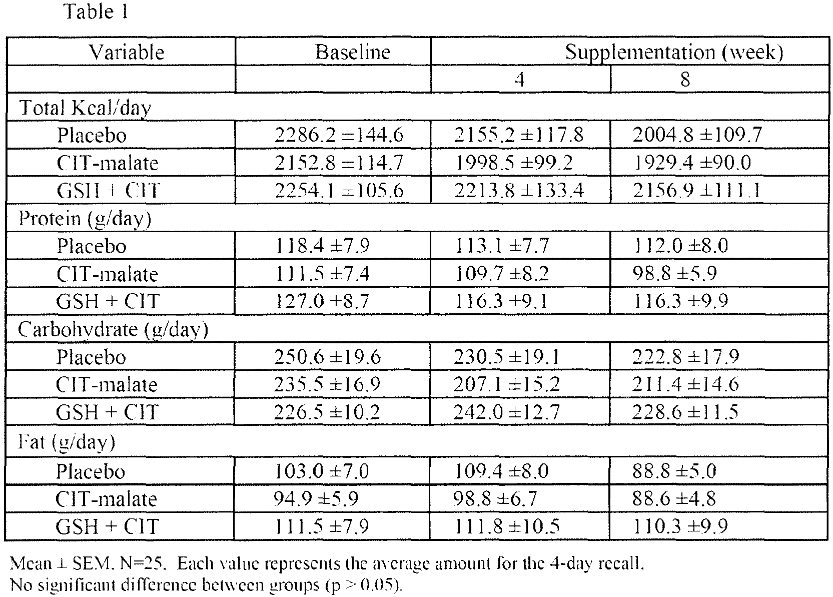

Each participant received a baseline assessment prior to beginning the study. The baseline assessment included measurement of body composition, muscle hypertrophy, and muscle strength, muscle power, and muscle endurance, as described herein.

Body composition was measured at days 0, 29, and 57 during the study. Total body mass (kg) was determined on a standard dual beam balance scale (Detecto. Bridgeview, IL). Percent body fat, fat mass, and fat-free mass were determined using DEXA (Discovery Series W. commercially available from Hologic, Waltham. MA). Quality control calibration procedures were performed on a spine phantom (X-CALIBER Model DPA/QDR- 1 anthropometric spine phantom, commercially available from Hologic. Waltham, MA) and a density step calibration phantom prior to each testing session. Total body water was determined with bioelectrical impedance spectroscopy (Tanita Inc., Arlington Heights, IL) using a low energy, high frequency current (500 micro amps at a frequency of 50 kHz). A summary of the participants' body compositions is set forth in Table 2.

### [Table 2]

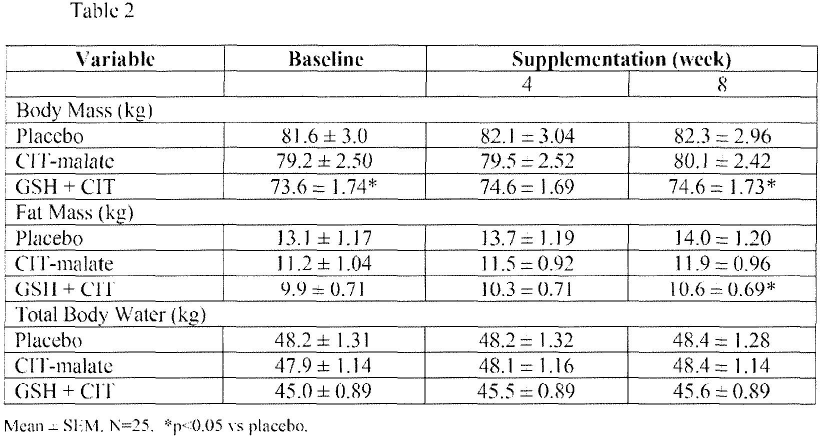

Muscle strength was evaluated using a 1-RM test (free-weight bench press and angled leg press exercises) on days 0, 29, and 57. as described herein. Participants warmed up by completing 5 to 10 repetitions at approximately 50% of the estimated 1-RM. Participants then rested for 1 minute, and then completed 3 to 5 repetitions at approximately 70% of the estimated 1-RM. The weight was then increased conservatively, and the participant attempted to lift the weight for one repetition. If the lift was successful, the participant rested for 2 minutes before attempting the next weight increment. This procedure was continued until the participant failed to complete the lift. The 1-RM was recorded as the maximum weight that the participant was able to lift for 1 repetition.

Participants engaged in a supervised, periodized 4 day per week resistance-training program split into two upper and two lower extremity workouts per week for a total of 8 weeks. Prior to the workout, participants performed a standardized series of stretching exercises. The participants then performed an upper-body resistance-training program consisting of bench press, lat pull, shoulder press, seated row, shoulder shrugs, chest fly, biceps curl, triceps press down, and abdominal curls, twice per week, and a lower-body program consisting of leg press, back extension, step ups, leg curls, leg extension, heel raises, and abdominal crunches, also performed twice per week. Participants performed 3 sets of 10 repetitions with as much weight as they could lift per set (typically 70 to 80% of 1-RM). Rest periods between exercises and sets lasted no longer than 2 minutes.

Venous blood samples were obtained from the antecubital vein using a standard VA-CUTAINER^{™} apparatus into one tube for serum separation and one for whole blood. The serum separation tubes were allowed to stand at room temperature for 15 minutes and then centrifuged for 10 minutes, and scrum was removed and placed into a microfuge tube. Blood samples were obtained prior to the first dose of supplement and prior to beginning the resistance training program (day 0) and after eight weeks of supplementation and resistance training (day 57).

Serum samples were assayed for the following general clinical chemistry markers: glucose, total protein, blood urea nitrogen, creatinine, BUN/creatinine ratio. AST, ALT, albumin, globulin, total bilirubin, alkaline phosphatase. Whole blood samples were assayed for standard cell blood counts with percentage differentials, such as hemoglobin, red blood cell counts, white blood cell counts, neutrophils, lymphocytes, monocytes, eosinophils, and basophils. The changes in blood biochemical and hematological markers during the study are set forth in Table 3.

### [Table 3]

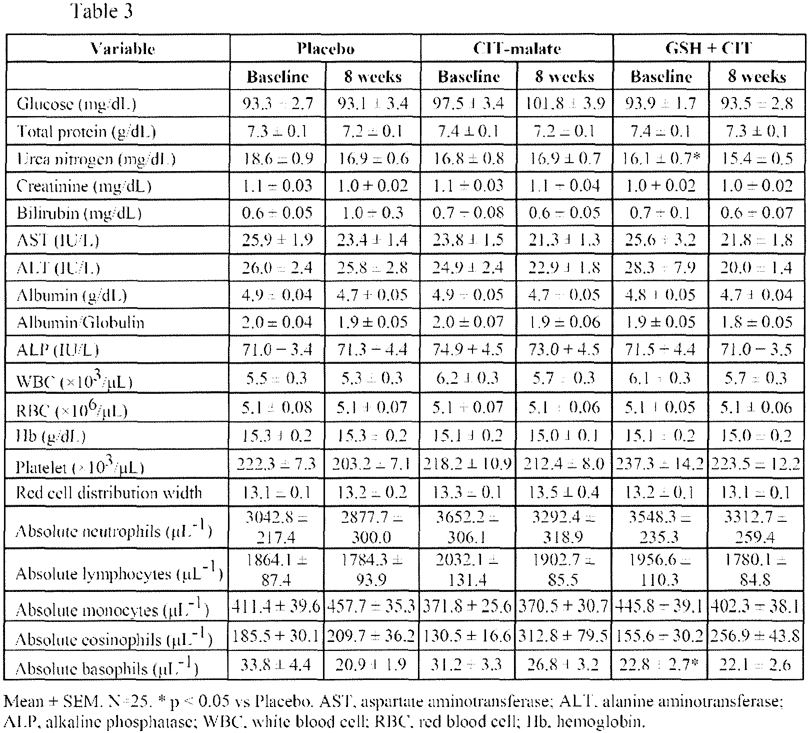

As is apparent from the data set forth in Table 3. none of the whole blood and serum clinical chemistry markers were negatively impacted in amongst any of the treatment groups. These results indicate that the oral ingestion of the supplements for a period of 8 weeks appears to be safe. In addition, none of the participants reported any adverse events associated with ingestion of the supplements.

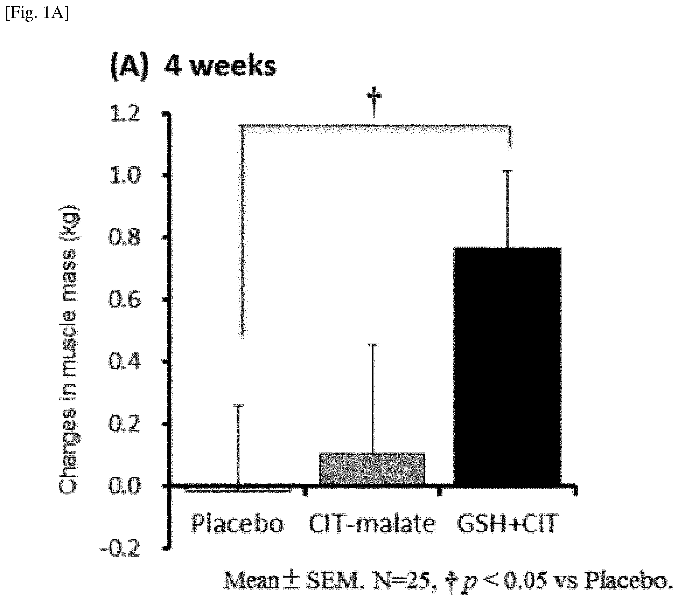

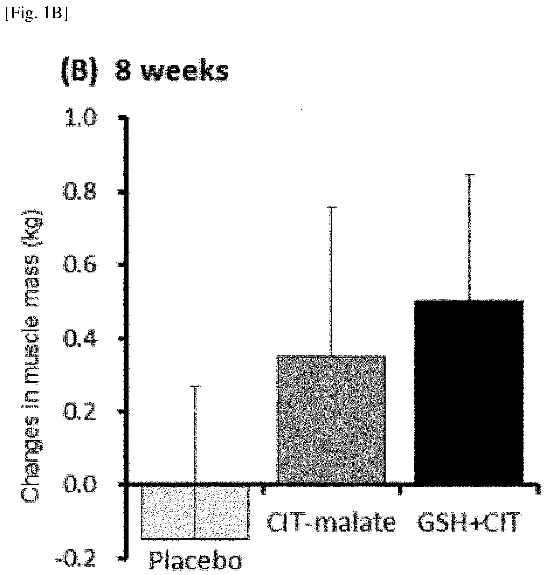

The data for changes in muscle mass at 4 and 8 weeks are set forth in in FIG. 1A (4 weeks) and FIG. 1B (8 weeks). As is apparent from the results set forth in FIGS. 1A and 1B. participants receiving either L-citrulline and glutathione, or L-citrulline malate exhibited an increase in muscle mass compared to persons receiving only placebo. Moreover, the increase in muscle mass in the test group administered L-citrulline and glutathione was greater than cither of the two groups, and the increase was statistically significant from placebo after 4 weeks (p < 0.05).

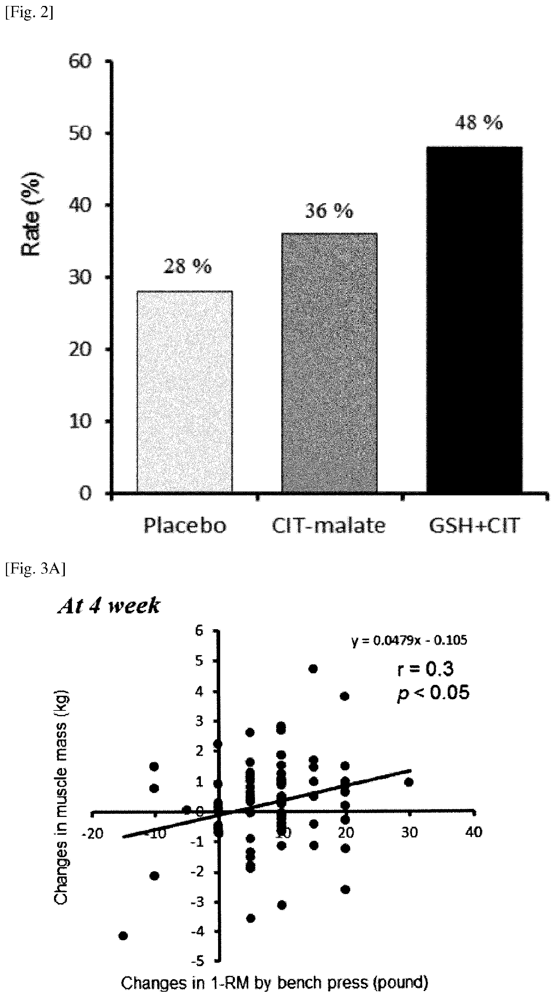

The data for the number of participants in cach group who displayed increases in muscle mass at both 4 and 8 weeks are set forth in FIG. 2. As depicted in FIG. 2, 48% of the participants in the L-citrulline and glutathione group exhibited an increase in muscle mass. In contrast, only 36% of the L-citrulline malate group and only 28% of the placebo group exhibited an increase in muscle mass. This data indicates that the combination of L-citrulline and glutathione was effective in increasing muscle mass in conjunction with resistance training.

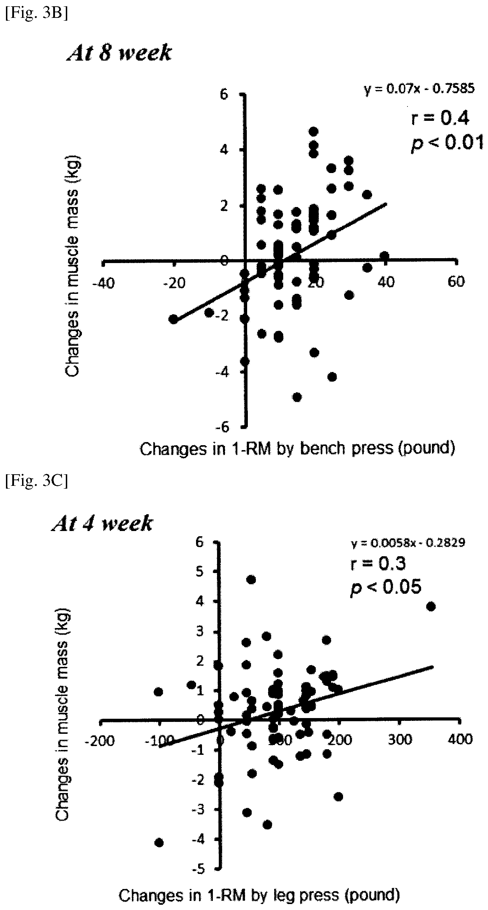

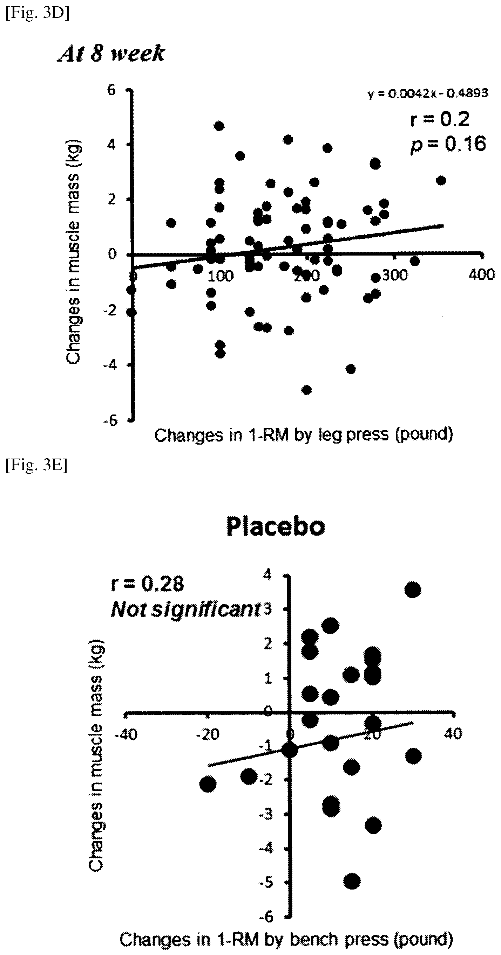

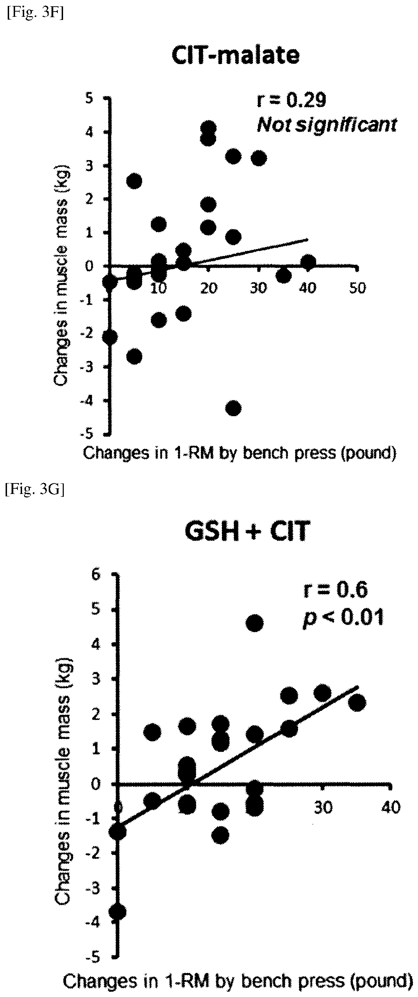

The relationship between muscle mass and muscle strength at weeks 4 and 8 for the bench press is presented in FIGS. 3A and 3B, respectively, and at weeks 4 and 8 for leg press exercises is presented in FIGS. 3C and 3D, respectively. In regard to muscle mass and bench press strength, there was a statistically significant relationship observed at week 4 (r = 0.3. p < 0.05) and week 8 (r = 0.4. p < 0.01). A statistically significant relationship for muscle mass and leg press strength was observed at week 4 (r=0.3, p < 0.05). These data indicate that increases in muscle mass are directly related to increases in muscle strength as a result of resistance training.

The relationship between changes in muscle mass and muscle strength (1-RM bench press) is presented in FIGS. 3E-3G. As depicted in FIG. 3G. a statistically significant correlation between muscle mass and muscle strength was observed in the subjects administered L-citrulline and glutathione.

The results of this example demonstrate that the combination of citrulline and glutathione increased muscle mass over placebo and citrulline-malate alone after 8 weeks, and that the increase for the combined administration of citrulline and glutathione over placebo was statistically significant. In addition, the increases in muscle mass for the combined administration of citrulline and glutathione were statistically correlated to the increases in muscle strength observed for the test subjects. Based on the fact that there were no significant changes over the course of the 8-week study for the dietary variables, and that the participants in the combined citrulline and glutathione group did not cat more total calories or protein than the other two groups, dietary intake can be ruled out as a possible confounding variable for the increases in muscle mass. Without wishing to be bound by any particular theory, it is believed that these increases in muscle mass the administration of both citrulline and glutathione occurred due to increases in muscle protein synthesis, which may be linked to nitric oxide-induced increases in cGMP.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e.. meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed clement as essential to the practice of the invcntion.

Preferred embodiments of this invention are described herein, including the best mode known to the inventors for carrying out the invention.

## Claims

1. A non-therapeutic method of increasing a ratio of muscle mass to total body weight in a person comprising
administering citrulline or a salt thereof and glutathione or a salt thereof in conjunction with a resistance workout program to a person, wherein
the citrulline or a salt thereof and glutathione or a salt thereof are administered daily for a period greater than 7 days,
the resistance workout program is administered for at least the entire period during which the citrulline or a salt thereof and glutathione or a salt thereof are administered, and
the muscle mass is measured by a body composition meter.

2. The non-therapeutic method of claim 1, wherein the amount of citrulline or salt thereof administered is about 300 mg/day or more, preferably
wherein the amount of citrulline or salt thereof administered is about 1 g/day or more, more preferably
wherein the amount of citrulline or salt thereof administered is about 2 g/day or more.

3. The non-therapeutic method of claim 1 or 2, wherein the amount of glutathione or a salt thereof administered is about 50 mg/day or more, preferably
wherein the amount of glutathione or a salt thereof administered is about 100 mg/day or more, more preferably
wherein the amount of glutathione or a salt thereof administered is about 200 mg/day or more.

4. The non-therapeutic method of any one of claims 1 to 3, wherein the amount of citrulline or salt thereof and the amount of glutathione or salt thereof administered each day is in a molar ratio of about 50:1 to about 1:1, preferably
wherein the amount of citrulline or salt thereof and the amount of glutathione or salt thereof administered each day is in a molar ratio of about 25:1 to about 1:1, more preferably
wherein the amount of citrulline or salt thereof and the amount of glutathione or salt thereof administered each day is in a molar ratio of about 10:1 to about 1:1, most preferably
wherein the amount of citrulline or salt thereof and the amount of glutathione or salt thereof administered each day is in a molar ratio of about 10:1.

5. The non-therapeutic method of any one of claims 1 to 4, wherein the citrulline or salt thereof and the glutathione or salt thereof are administered daily for a period of at least 14 days, preferably
wherein the citrulline or salt thereof and glutathione or salt thereof are administered daily for a period of at least 21 days, more preferably
wherein the citrulline or salt thereof and glutathione or salt thereof are administered daily for a period of at least 28 days.

6. The non-therapeutic method of any one of claims 1 to 5, wherein the resistance workout program comprises a daily resistance workout of 4 days per week, and wherein the daily resistance workout comprises at least 8 repetitions of an exercise that targets a muscle of interest.

7. The non-therapeutic method of any one of claims 1 to 6, wherein the citrulline or salt thereof and glutathione or salt thereof are administered about 1 hour before a resistance workout.

8. The non-therapeutic method of any one of claims 1 to 6, wherein the citrulline or salt thereof and glutathione or salt thereof are administered within about 1 hour or less after a resistance workout.

9. The non-therapeutic method of any one of claims 1 to 8, wherein the ratio of muscle mass to total body weight is increased by about 0.3% or more, preferably
wherein the ratio of muscle mass to total body weight is increased by about 0.6% or more, more preferably
wherein the ratio of muscle mass to total body weight is increased by about 1% or more.

10. The non-therapeutic method of any one of claims 1 to 9, wherein the citrulline or salt thereof and glutathione or salt thereof are administered concurrently.

11. The non-therapeutic method of any one of claims 1 to 10, wherein the citrulline or salt thereof and glutathione or salt thereof are administered orally.

12. The non-therapeutic method of any one of claims 1 to 11, wherein the citrulline is L-citrulline.

## Patentansprüche

1. Ein nicht-therapeutisches Verfahren zum Erhöhen eines Verhältnisses von Muskelmasse zu Gesamtkörpergewicht bei einer Person, umfassend
Verabreichen von Citrullin oder einem Salz davon und Glutathion oder einem Salz davon in Verbindung mit einem Krafttrainingsprogramm an eine Person, wobei das Citrullin oder ein Salz davon und das Glutathion oder ein Salz davon täglich über einen Zeitraum von mehr als 7 Tagen verabreicht werden,
das Krafttrainingsprogramm für mindestens den gesamten Zeitraum durchgeführt wird, in dem das Citrullin oder ein Salz davon und das Glutathion oder ein Salz davon verabreicht werden, und
die Muskelmasse mit einem Gerät zur Messung der Körperzusammensetzung gemessen wird.

2. Das nicht-therapeutische Verfahren nach Anspruch 1, wobei die verabreichte Menge an Citrullin oder einem Salz davon etwa 300 mg/Tag oder mehr beträgt, vorzugsweise
wobei die verabreichte Menge an Citrullin oder einem Salz davon etwa 1 g/Tag oder mehr beträgt, stärker bevorzugt
wobei die verabreichte Menge an Citrullin oder einem Salz davon, etwa 2 g/Tag oder mehr beträgt.

3. Das nicht-therapeutische Verfahren nach Anspruch 1 oder 2, wobei die verabreichte Menge an Glutathion oder einem Salz davon etwa 50 mg/Tag oder mehr beträgt, vorzugsweise
wobei die verabreichte Menge an Glutathion oder einem Salz davon etwa 100 mg/Tag oder mehr beträgt, stärker bevorzugt
wobei die verabreichte Menge an Glutathion oder einem Salz davon etwa 200 mg/Tag oder mehr beträgt.

4. Das nicht-therapeutische Verfahren nach einem der Ansprüche 1 bis 3, wobei die Menge an Citrullin oder einem Salz davon und die Menge an Glutathion oder einem Salz davon, die täglich verabreicht werden, ein Molverhältnis von etwa 50:1 bis etwa 1:1 aufweist, vorzugsweise
wobei die Menge an Citrullin oder einem Salz davon und die Menge an Glutathion oder einem Salz davon, die täglich verabreicht werden, ein Molverhältnis von etwa 25:1 bis etwa 1:1 aufweist, stärker bevorzugt
wobei die Menge an Citrullin oder einem Salz davon und die Menge an Glutathion oder einem Salz davon, die täglich verabreicht werden, ein Molverhältnis von etwa 10:1 bis etwa 1:1 aufweist, stärker bevorzugt
wobei die Menge an Citrullin oder einem Salz davon und die Menge an Glutathion oder einem Salz davon, die täglich verabreicht werden, ein Molverhältnis von etwa 10: 1 aufweist.

5. Das nicht-therapeutische Verfahren nach einem der Ansprüche 1 bis 4, wobei das Citrullin oder ein Salz davon und das Glutathion oder ein Salz davon täglich über einen Zeitraum von mindestens 14 Tage verabreicht werden, vorzugsweise
wobei das Citrullin oder ein Salz davon und das Glutathion oder ein Salz davon täglich über einen Zeitraum von mindestens 21 Tagen verabreicht werden, stärker bevorzugt
wobei das Citrullin oder ein Salz davon und das Glutathion oder ein Salz davon täglich über einen Zeitraum von mindestens 28 Tagen verabreicht werden.

6. Das nicht-therapeutische Verfahren nach einem der Ansprüche 1 bis 5, wobei das Krafttrainingsprogramm ein tägliches Krafttraining an 4 Tagen pro Woche umfasst und wobei das tägliche Krafttraining mindestens 8 Wiederholungen einer Übung umfasst, die auf einen bestimmten Muskel abzielt.

7. Das nicht-therapeutische Verfahren nach einem der Ansprüche 1 bis 6, wobei das Citrullin oder ein Salz davon und das Glutathion oder ein Salz davon etwa 1 Stunde vor einem Krafttraining verabreicht werden.

8. Das nicht-therapeutische Verfahren nach einem der Ansprüche 1 bis 6, wobei das Citrullin oder ein Salz davon und das Glutathion oder ein Salz davon innerhalb von etwa 1 Stunde oder weniger nach einem Krafttraining verabreicht werden.

9. Das nicht-therapeutische Verfahren nach einem der Ansprüche 1 bis 8, wobei das Verhältnis von Muskelmasse zu Gesamtkörpergewicht um etwa 0,3 % oder mehr erhöht wird, vorzugsweise
wobei das Verhältnis von Muskelmasse zu Gesamtkörpergewicht um etwa 0,6 % oder mehr erhöht wird, stärker bevorzugt
wobei das Verhältnis von Muskelmasse zu Gesamtkörpergewicht um etwa 1 % oder mehr erhöht wird.

10. Das nicht-therapeutische Verfahren nach einem der Ansprüche 1 bis 9, wobei das Citrullin oder ein Salz davon und das Glutathion oder ein Salz davon gleichzeitig verabreicht werden.

11. Das nicht-therapeutische Verfahren nach einem der Ansprüche 1 bis 10, wobei das Citrullin oder ein Salz davon und das Glutathion oder ein Salz davon oral verabreicht werden.

12. Das nicht-therapeutische Verfahren nach einem der Ansprüche 1 bis 11, wobei das Citrullin L-Citrullin ist.

## Revendications

1. Méthode non thérapeutique d'augmentation du rapport de la masse musculaire au poids corporel total chez une personne, comprenant
l'administration à une personne de citrulline ou d'un sel de celle-ci et de glutathion ou d'un sel de celui-ci conjointement avec un programme d'entraînement de résistance, dans laquelle
la citrulline ou un sel de celle-ci et le glutathion ou un sel de celui-ci sont administrés quotidiennement pendant une période supérieure à 7 jours,
le programme d'entraînement de résistance est administré pendant au moins toute la période durant laquelle la citrulline ou un sel de celle-ci et le glutathion ou un sel de celui-ci sont administrés, et
la masse musculaire est mesurée par un dispositif de mesure de composition corporelle.

2. Méthode non thérapeutique selon la revendication 1, dans laquelle la quantité de citrulline ou de sel de celle-ci administrée est d'environ 300 mg/jour ou plus, de préférence
dans laquelle la quantité de citrulline ou de sel de celle-ci administrée est d'environ 1 g/jour ou plus, plus préférentiellement
dans laquelle la quantité de citrulline ou de sel de celle-ci administrée est d'environ 2 g/jour ou plus.

3. Méthode non thérapeutique selon la revendication 1 ou 2, dans laquelle la quantité de glutathion ou d'un sel de celui-ci administrée est d'environ 50 mg/jour ou plus, de préférence
dans laquelle la quantité de glutathion ou d'un sel de celui-ci administrée est d'environ 100 mg/jour ou plus, plus préférentiellement
dans laquelle la quantité de glutathion ou d'un sel de celui-ci administrée est d'environ 200 mg/jour ou plus.

4. Méthode non thérapeutique selon l'une quelconque des revendications 1 à 3, dans laquelle la quantité de citrulline ou de sel de celle-ci et la quantité de glutathion ou de sel de celui-ci administrées chaque jour sont en un rapport molaire d'environ 50/1 à environ 1/1, de préférence
dans laquelle la quantité de citrulline ou de sel de celle-ci et la quantité de glutathion ou de sel de celui-ci administrées chaque jour sont en un rapport molaire d'environ 25/1 à environ 1/1, plus préférentiellement
dans laquelle la quantité de citrulline ou de sel de celle-ci et la quantité de glutathion ou de sel de celui-ci administrées chaque jour sont en un rapport molaire d'environ 10/1 à environ 1/1, encore plus préférentiellement
dans laquelle la quantité de citrulline ou de sel de celle-ci et la quantité de glutathion ou de sel de celui-ci administrées chaque jour sont en un rapport molaire d'environ 10/1.

5. Méthode non thérapeutique selon l'une quelconque des revendications 1 à 4, dans laquelle la citrulline ou le sel de celle-ci et le glutathion ou le sel de celui-ci sont administrés quotidiennement pendant une période d'au moins 14 jours, de préférence
dans laquelle la citrulline ou le sel de celle-ci et le glutathion ou le sel de celui-ci sont administrés quotidiennement pendant une période d'au moins 21 jours, plus préférentiellement
dans laquelle la citrulline ou le sel de celle-ci et le glutathion ou le sel de celui-ci sont administrés quotidiennement pendant une période d'au moins 28 jours.

6. Méthode non thérapeutique selon l'une quelconque des revendications 1 à 5, dans laquelle le programme d'entraînement de résistance comprend un entraînement de résistance quotidien 4 jours par semaine, et dans laquelle le programme d'entraînement de résistance comprend au moins 8 répétitions d'un exercice qui cible un muscle d'intérêt.

7. Méthode non thérapeutique selon l'une quelconque des revendications 1 à 6, dans laquelle la citrulline ou le sel de celle-ci et le glutathion ou le sel de celui-ci sont administrés environ 1 heure avant un entraînement de résistance.

8. Méthode non thérapeutique selon l'une quelconque des revendications 1 à 6, dans laquelle la citrulline ou le sel de celle-ci et le glutathion ou le sel de celui-ci sont administrés environ 1 heure ou moins après un entraînement de résistance.

9. Méthode non thérapeutique selon l'une quelconque des revendications 1 à 8, dans laquelle le rapport de la masse musculaire au poids corporel total est augmenté d'environ 0,3 % ou plus, de préférence
dans laquelle le rapport de la masse musculaire au poids corporel total est augmenté d'environ 0,6 % ou plus, plus préférentiellement
dans laquelle le rapport de la masse musculaire au poids corporel total est augmenté d'environ 1 % ou plus.

10. Méthode non thérapeutique selon l'une quelconque des revendications 1 à 9, dans laquelle la citrulline ou le sel de celle-ci et le glutathion ou le sel de celui-ci sont administrés en même temps.

11. Méthode non thérapeutique selon l'une quelconque des revendications 1 à 10, dans laquelle la citrulline ou le sel de celle-ci et le glutathion ou le sel de celui-ci sont administrés par voie orale.

12. Méthode non thérapeutique selon l'une quelconque des revendications 1 à 11, dans laquelle la citrulline est la L-citrulline.
